# EUROPEAN PATENT SPECIFICATION

(11) **EP 2 055 323 B1**
(45) Date of publication and mention of the grant of the patent: **20.03.2013**
(21) Application number: 08253488.4
(22) Date of filing: 27.10.2008
(51) Int. Cl.: A61L 17/14, A61L 31/14

(54) **Filament-reinforced composite fiber**
Fadenverstärkte Verbundstofffaser
Filament renforcé par des fibres composites

(30) Priority: 29.10.2007 US 983262 P; 15.10.2008 US 251909
(43) Date of publication of application: 06.05.2009
(73) Proprietor: Covidien LP, Mansfield, MA 02048 (US)
(72) Inventor: Stopek, Joshua, Yalesville, CT 06492 (US)
(74) Representative: Soames, Candida Jane

(56) References cited:
- EP-A2- 1 917 983
- US-A- 5 569 302
- US-A1- 2004 060 409
- US-B1- 6 264 675

## Description

### BACKGROUND

### Technical Field

The present disclosure relates generally to the field of surgical devices, and more particularty to filament-reinforced composites useful in forming surgical devices including implantable materials such as fibers, mesh, and sutures.

### Background of Related Art

Surgical sutures have been successfully used for various types of medical procedures, including tissue and wound closure. Surgical sutures are available in various sizes and types (e.g. monofilament and braided) to support different types of tissue closure. Surgical sutures typically have a needle attached at one end. As the needle penetrates tissue, the suture enters, passes through, and exits the tissue, at which point knots, may be used to secure the tissue or wound.

Monofilament barbed sutures (commercially available from Surgical Specialties) have been approved for use in cosmetic ptosis procedures, including brow and face lifts. Barbs along the length of the suture engage the tissue to secure the barbed suture in place without the need for knots. High loads and stresses are imparted to a single filament during the barb formation process. These stresses may weaken the filament and barbs may be easily peeled away from the filament, especially in higher tension wounds.

US 6 264 675 and EP 1 917 983 disclose a fibre in accordance with the preamble of claim 1.

### SUMMARY

In a first aspect of the present'disclosure, a filament-reinforced composite fiber in accordance with claim 1 includes a plurality of filaments, an adhesive matrix material, and the filament-reinforced composite fibers having at least one integral barb formed on a surface thereof. In embodiments, the filament-reinforced composite fiber may be used as a suture or incorporated into a mesh.

One method for making a medical device in accordance with claim 21 includes applying an adhesive matrix material to a plurality of filaments to provide a filament-reinforced composite fiber, and creating at least one barb on a surface of the filament-reinforced composite fiber.

### BRIEF DESCRIPTION OF DRAWINGS

Various preferred embodiments of the filament-reinforced composite fibers are described herein with reference to the drawings, in which:

FIG. 1 is a plan view illustrating an embodiment of a filament-reinforced composite fiber formed into a mesh which includes barbs;

FIG. 2 is a side view illustrating one embodiment of a filament-reinforced composite fiber suture;

FIGS. 3A-3D are partial views illustrating one embodiment of a filament-reinforced composite fiber;

FIG. 4 illustrates a method according to an embodiment of forming barbs on a filament-reinforced composite fiber; and,

FIG. 5 is a side view illustrating a further embodiment of a filament-reinforced composite fiber suture having a bidirectional barbed structure.

### DETAILED DESCRIPTION OF EMBODIMENTS

The present disclosure is directed to filament-reinforced composite fibers in accordance with claim 1. The filament-reinforced composite fiber has an elongated body, formed from a plurality of filaments, in combination with an adhesive matrix material. Medical devices of embodiments of the present disclosure include surgical meshes and surgical sutures made from the filament-reinforced composite fiber.

A "filament" as described means a continuous strand that has an elongate body portion, with a ratio of length to diameter greater than 10:1. The term "filament" is used herein in describing a plurality of "filaments" which total or combine to create a "fiber." In embodiments, several filaments may be knitted, woven, fused or braided together, creating a fiber.

Generally, filaments may have a diameter of from about 1 micron to about 50 microns, in embodiments, from about 5 microns to about 15 microns. Individual filaments suitable for forming the filament-reinforced composite fiber may be derived from any fiber-forming synthetic or natural material, for example, polymers and metals (e.g., magnesium derivatives, steel and titanium). The fiber-forming materials may be bioabsorbable or non-bioabsorbable. It should be understood that combinations of filaments made from different materials (e.g. natural and synthetic, or bioabsorbable and non-bioabsorbable materials) may be used to make the present filament-reinforced composite fiber.

Suitable synthetic absorbable materials include polymers such as those made from lactide, glycolide, caprolactone, valerolactone, carbonates (e.g., trimethylene carbonate, tetramethylene carbonate, and the like), dioxanones (e.g., 1,4-dioxanone) δ-valerolactone, 1,dioxepanones (e.g., 1,4-dioxepan-2-one and 1,5-dioxepan-2-one), ethylene glycol, ethylene oxide, esteramides, γ-hydroxyvalerate, β-hydroxypropionate, alpha-hydroxy acid, hydroxybuterates, orthoesters, hydroxy alkanoates, tyrosine carbonates, polyimide carbonates, polyimino carbonates such as poly (bisphenol A-iminocarbonate) and poly (hydroquinone-iminocarbonate), and polymer drugs (e.g., polydiflunisol, polyaspirin, and protein therapeutics) and copolymers and combinations thereof. Suitable natural absorbable polymers include collagen, cellulose and gut. In embodiments, glycolide and lactide based polyesters, including copolymers of lactide and glycolide may be used.

Suitable non-absorbable materials which may be used to form filament-reinforced composite fibers include non-absorbable natural materials such as cotton, silk, and rubber. Suitable non-absorbable synthetic materials include monomers and polymers derived from materials such as nylons, polyolefins such as polypropylene and polyethylene, ultra high molecular weight polyethylene (UHMWPE), polyamides, polyesters such as poly ethylene terepththalate (PET), polyaryletherketone, polyvinylidene difluoride (PVDF), acrylic, polyamides, aramids, fluropolymers, polybutesters, silicones, and polymer blends, copolymers thereof and combinations with degradable polymers. In embodiments, polypropylene can be utilized to form the suture. The polypropylene can be isotactic polypropylene or a mixture of isotactic and syndiotactic or atactic polypropylene. Additionally, non-absorbable synthetic and natural polymers and monomers may be combined with each other and may also be combined with various absorbable polymers and monomers to create fibers and filaments for the present filament-reinforced composite fiber.

In embodiments, filaments may be constructed using shape memory polymers. Suitable polymers used to prepare hard and soft segments of shape memory polymers include polycaprolactone, dioxanone, lactide, glycolide, polyacrylates, polyamides, polysiloxanes, polyurethanes, polyether amides, polyurethane/ureas, polyether esters, and urethane/butadiene copolymers and combinations thereof.

In embodiments, the filaments may be formed of metals (e.g. steel and degradable magnesium), metal alloys or the like.

The term "filament" is used herein in describing a plurality of "filaments" which total or combine to create a "fiber." Methods for making filaments from these suitable materials are within the purview of those skilled in the art (e.g. extrusion and molding). The plurality of filaments can be combined in any manner for use in the present disclosure. The plurality of filaments may be combined using any technique within the purview of one skilled in the art such as commingling, twisting, braiding, weaving, entangling, and knitting. For example, a plurality of filaments may simply be combined to form a yarn. As another example, a plurality of filaments may be braided. As yet another example, a plurality of filaments may be combined to form a yarn and then those multifilament yarns may be braided. Those skilled in the art reading this disclosure will envision other ways in which filaments may be combined. Filaments may also be combined to produce a non-woven multifilament fiber. In certain embodiments, a multifilament structure useful in forming a filament-reinforced composite fiber according to the present disclosure may be produced by braiding. The braiding can be done by any method within the purview of those skilled in the art. For example, braid constructions for sutures and other medical devices are described in U.S. Patent Nos. 5,019,093 5,059,213 5,133,738 5,181,923 5,226,912 5,261,886 5,306,289 5,318,575 5,370,031 5,383,387 5,662,682 5,667,528, and 6,203,564.

In some cases a tubular braid or sheath can be constructed about a core structure which is fed through the center of a braider. Known tubular braided sutures, including those possessing cores, are disclosed, for example in U.S. Patent Nos. 3,187,752, 3,565,077, 4,104,973, 4,043,344, and 4,047,533.

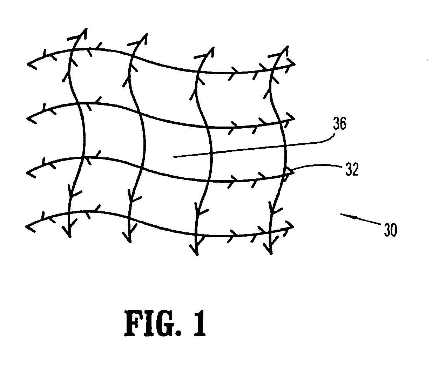

Suitable filaments may be drawn, braided, knitted, oriented, crinkled, twisted, entangled, commingled or substantially parallel to form yarns as part of a fiber-forming process. Additionally, the present filament-reinforced composite fibers, such as mesh, may be formed by braiding, weaving, knitting, matting, spraying, etc., individual filaments together. FIG. 1 shows one embodiment, a mesh 30 formed from filament-reinforced composite fibers of the present disclosure. The mesh 30 has barbs 32 along a portion of the fiber-reinforced composite fiber, whereas the center of the mesh includes an unbarbed portion 36. By applying an adhesive material to the plurality of filaments, a filament-reinforced composite fiber can be constructed. Suitable matrix materials include any compound or composition that can penetrate or be caused to penetrate between the individual filaments and/or into interstices between individual filaments, such that the filaments are embedded within the matrix material, bound together, creating a coherent composite material. In embodiments where barbs are formed in the filament-reinforced composite fiber, the matrix penetrates the plurality of filaments deeper than the depth of the barb to provide support for the regions to be barbed.

Suitable matrix materials include but are not limited to absorbable and non-absorbable materials including cyanoacrylates, isocyanates, polyurethanes, polyamines, polyamides, polyacrylates, polymethacrylates, silicones, carbonates, and other synthetic monomers and polymers and combinations thereof. Matrix materials may also include natural materials such as fibrins, albumins, thrombin, gelatin, proteins, collagens, polysaccharides, and combinations thereof.

In embodiments, matrix materials such as cyanoacrylates can be employed in the present disclosure. Suitable cyanoacrylates include materials derived from methyl cyanoacrylate, ethyl cyanoacrylate, butyl cyanoacrylate, octyl cyanoacrylate, isobutyl cyanoacrylate and methoxypropyl cyanoacrylate and combinations thereof and the like.

In embodiments, suitable adhesive matrix materials include synthetic absorbable and non-absorbable monomers and oligomers including those synthesized from materials such as lactic acid, glycolic acid, caprolactone, dioxanone, PEG, pluronics, isocyanates, and combinations thereof and the like.

Adhesive matrix materials may also be combined with solvents, including polar and nonpolar solvents. Suitable solvents include alcohols, e.g., methanol, ethanol, propanol, chlorinated hydrocarbons (such as methylene chloride, chloroform, 1, 2-dichloro-ethane), and aliphatic hydrocarbons such as hexane, heptene, ethyl acetate.

The matrix material can be applied at regular or irregular intervals. For example, the matrix material can be applied along a plurality of filaments only where barbs are to be present along the filament-reinforced composite fiber as described in more detail below. In other embodiments, the matrix materials can be applied along the length of the entire fiber. In another embodiment, a suture may include the filament-reinforced composite fiber on a first portion, while a second portion includes only a plurality of filaments. This may be desirable when different suture strengths are required, for example, using one fiber to close various tissue types and layers. Generally, the matrix may be applied on at least 5% of the length of the plurality of filaments. In embodiments, the matrix material is present on about 5% to about 95%, in embodiments about 20% to about 70% of the total length of the plurality of filaments used to make the present filament-reinforced composite fiber.

Matrix materials may be applied to the plurality of filaments using any technique within the purview of one skilled in the art such as dipping, spraying, brushing, vapor deposition, coextrusion, capillary wicking, film casting, molding and the like. The matrix material may be applied following or during manipulation (e.g., braiding, weaving, or knitting, etc.) of the plurality of filaments. In embodiments, the matrix material may be applied to individual filaments immediately prior to weaving or braiding the filaments, creating a filament-reinforced composite fiber.

Additionally, the composite may include biologically acceptable additives such as plasticizers, antioxidants, dyes, dilutants, bioactive agents and combinations thereof, which can be coated on the filaments or fibers, or impregnated into the fibers or filaments and/or adhesive matrix which are used to form the composite of the present disclosure.

Various compositions and materials may optionally also be applied to the filaments, fibers, and/or included in the matrix materials to improve mechanical properties such as handling and knot strength or to deliver medicinal agents. Suitable coating materials include any materials conventionally applied to sutures. For example, suitable materials include fatty acid esters which may be combined with the metal salt of a fatty acid in the coating composition. Such esters include, for example, calcium stearate, stearoyl lactylate esters, palmityl lactylate esters, oleyl lactylate esters such as calcium, magnesium, aluminum, barium, or zinc stearoyl lactylate, calcium, magnesium, aluminum, barium, or zinc palmityl lactylate; calcium, magnesium, aluminum, barium, or zinc oleyl lactylate; with calcium stearate and calcium stearoyl-2-lactylate (such as the calcium stearoyl-2-lactylate commercially available under the trade name VERV from American Ingredients Co., Kansas City, Mo.) being preferred. When desirable, the fatty acid ester may be combined with a solvent. Suitable solvents include those listed above.

In embodiments, the filament-reinforced composite fibers may be combined with and/or coated with suitable materials including polyalkylene oxides such as polyethylene oxide, polypropylene oxide, polyethylene glycol (PEG), polypropylene glycol, copolymers thereof, and the like, including those having acrylate groups such as acrylate PEGs, and acrylate PEG/PPG copolymers. Such combinations may include blends or copolymers with polyalkylene oxide oligomers or polymers or other non-toxic surfactants. The resulting composition may possess antimicrobial properties due to the presence of the copolymers described above. In other embodiments, the filament-reinforced composite fibers may be combined with silicone acrylates. Coatings may be applied to the individual filaments or the composite fiber structure at any time prior to sterilization techniques. Coatings can be applied to the filaments or the composite fiber using any technique within the purview of those skilled in the art.

Additionally, the filament-reinforced composite fiber may incorporate various pharmaceuticals and medicinal agents. Braided filaments have a high surface area, including several individual filaments and interstitial sites (between the filaments) available for drug loading and storage depots. Bulk loading of various bioactive/medicinal agents can be achieved, and optionally sealed into the braid once the matrix is applied, for controlled release/elution over a desired time course. Medicinal agents and drugs may be applied to the filaments and/or fiber by methods within the purview of those skilled in the art, including but not limited to dipping, spraying, brushing, vapor deposition, coextrusion, capillary wicking, film casting, molding and the like. Suitable agents may also be incorporated into the adhesive matrix by mixing, stirring, emulsions, phase separations and the like. Additionally, solvents may be used to incorporate various agents into the composite device. Suitable solvent include those listed above.

Medicinal agents which may be incorporated into the composite include antimicrobial agents, anti-virals, anti-fungals, and the like. Antimicrobial agents as used herein is defined by an agent which by itself or through assisting the body (immune system) helps the body destroy or resist microorganisms which may be pathogenic (disease causing). The term "antimicrobial agent" includes antibiotics, quorum sensing blockers, surfactants, metal ions, antimicrobial proteins and peptides, antimicrobial polysaccharides, antiseptics, disinfectants, anti-virals, anti-fungals, and combinations thereof.

Examples of suitable antiseptics and disinfectants which may be combined with the present disclosure include hexachlorophene, cationic biguanides like chlorohexadine and cyclohexidine, iodine and iodophores like povidone-iodine, halo-substituted phenolic compounds like PCMX (i.e., p-chloro-m-xylenon) and triclosan (e.g., 2,4,4'-trichloro-2'hydroxydiphenylether), furan medical preparations like nitrofurantoin and nitrofurazone, methanamine, aldehydes like gluteraldehyde and formaldehyde, alcohols, combinations thereof, and the like. In some embodiments, at least one of the antimicrobial agents may be an antiseptic, such as triclosan.

Classes of antibiotics that can be combined with the present disclosure include tetracyclines like minocycline, rifamycins like rifampin, macrolides like erythromycin, penicillins like nafcillin, cephalosporins like cefazolon, beta-lactam antibiotics like imipenen and aztreonam, aminoglycosides like gentamicin and TOBRAMYCIN®, chloramphenicol, sulfonamides like sulfamethoxazole, glycopeptides like vancomycin, quilones like ciproflaxin, fusidic acid, trimethoprim, metronidazole, clindamycin, mupirocin, polyenes like amphotericin B, azoles like fluconazole, and beta-lactam inhibitors like sublactam. Other antimicrobials which may be added include, for example antimicrobial-peptides and/or proteins, antimicrobial polysaccharides, quorum sensing blockers, anti-virals, metal ions such as ionic silver and ionic silver glass, surfactants, chemotherapeutic drug, telomerase inhibitors, other cyclic monomers including 5-cyclic monomers, mitoxantrone, and the like.

Additional suitable medicinal agents which may be used include colorants, dyes, preservatives, protein and peptide preparations, protein therapeutics, polysaccharides such as hyaluronic acid, lectins, lipids, probiotics, antibiotics, angiogenic agents, anti-thrombotics, anticlotting agents, clotting agents, analgesics, anesthetics, wound repair agents, chemotherapeutics, biologics, anti-inflammatory agents, anti-proliferatives, diagnostic agents, antipyretic, antiphlogistic and analgesic agents, vasodilators, antihypertensive and antiarrhythmic agents, hypotensive agents, antitussive agents, antineoplastics, local anesthetics, hormone preparations, antiasthmatic and antiallergic agents, antihistaminics, anticoagulants, antispasmodics, cerebral circulation and metabolism improvers, antidepressant and antianxiety agents, vitamin D preparations, hypoglycemic agents, antiulcer agents, hypnotics, antibiotics, antifungal agents, sedative agents, bronchodilator agents, antiviral agents, dysuric agents, brominated or halogenated furanones, and the like. In embodiments, polymer drugs, i.e., polymeric forms of such compounds for example, polymeric antibiotics, polymeric antiseptics, polymeric chemotherapeutics, polymeric anti-proliferatives, polymeric antiseptics, polymeric non-steroidal anti-inflammatory drugs (NSAIDS), and the like may be utilized and combinations thereof.

The filament-reinforced composite fiber of the present disclosure can additionally contain suitable medicinal agents such as viruses and cells, peptides, polypeptides and proteins, analogs, muteins, and active fragments thereof, such as immunoglobulins, antibodies (monoclonal and polyclonal), cytokines (e.g. lymphokines, monokines, chemokines), blood clotting factors, hemopoietic factors, interleukins (IL-2, IL-3, IL-4, IL-6), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.) hormones and hormone analogs (e.g., growth hormone), vaccines (e.g., tumoral, bacterial and viral antigens), somatostatin, antigens, blood coagulation factors, growth factors, protein inhibitors, protein antagonists, and protein agonists, nucleic acids, such as antisense molecules, DNA, RNA, oligonucleotides, polynucleotides and ribozymes and combinations thereof.

Methods for combining these medicinal agents with compositions of the present disclosure are within the purview of those skilled in the art and include, but are not limited to mixing, blending, dipping, spraying, wicking, solvent evaporating and the like.

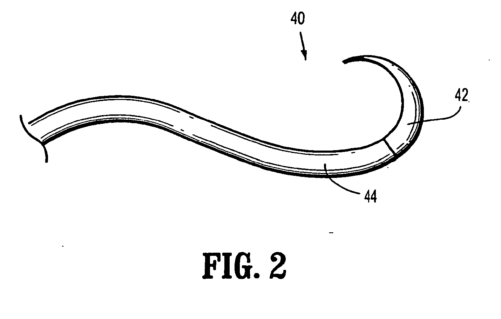

In embodiments, the present filament-reinforced composite fiber is used as a suture. As shown in FIG. 2, a filament-reinforced composite fiber 40 may include a needle 42 disposed at one end of the suture 44. Sutures typically have good handling properties, including the ability to tie a knot therein. Handling properties may be evaluated using standard tests such as knot tying, knot security, knot reposition and knot run down characteristics, the testing of which can be may be quantitative or qualitative in nature and vary by suture composition and processing constraints. For example, a bench top tie board and/or *in vivo* animal evaluations may be used to determine a suture's handling properties such as knot rundown, knot reposition and security characteristics. Filament-reinforced composite fibers also provide a higher resistance to peel strength. In embodiments to be discussed later, a barbed filament-reinforced composite fiber would have a high barb peel strength, which enables the barbed composite to hold higher tension wounds. Peel strength may be tested qualitatively or quantitatively and values assigned.

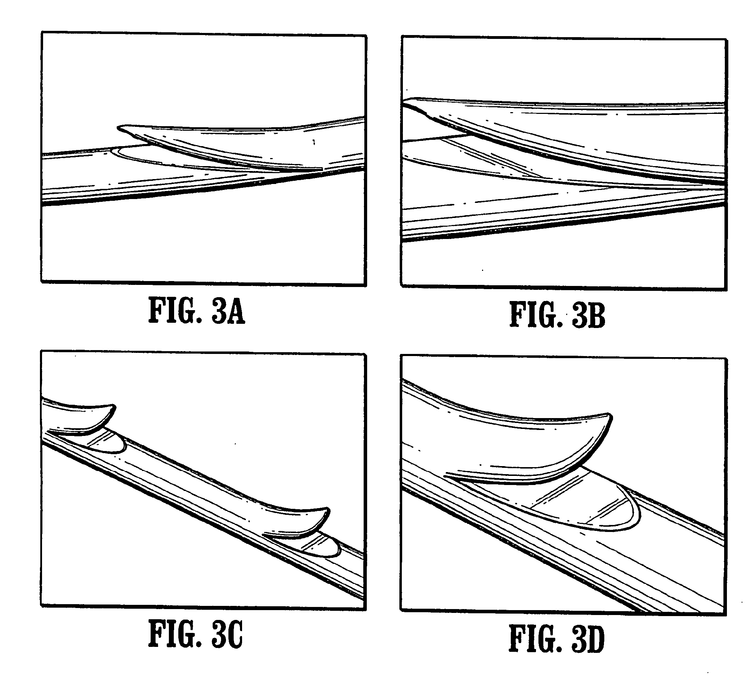

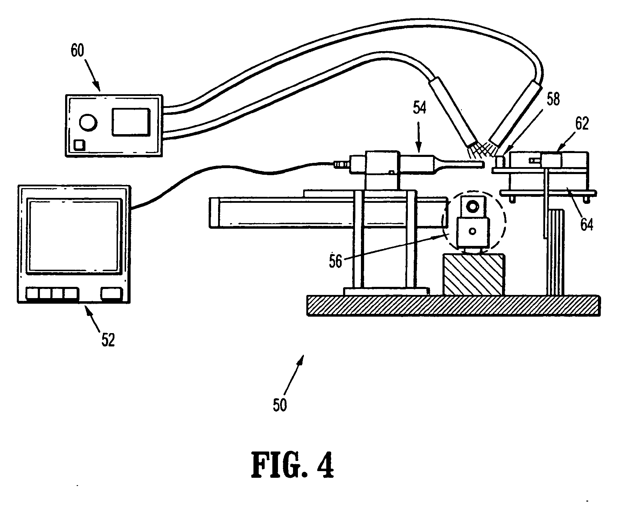

The filament-reinforced composite fibers include barbs, which are formed on the surface thereon and are integral to the composite. FIG. 3 shows a barb 12 on the disclosed filament-reinforced composite fiber 10. Barbs can be created on the filament-reinforced composite fiber using any technique, including but not limited to lasers, molding, knives, blades, stamping, and other cutting means within the purview of those skilled in the art. In embodiments, ultrasonic energy can also be used to create barbs as described in U.S. Patent Application No. 60/994,173 filed on September 17, 2007 entitled "Method of Forming Barbs on a Suture". FIG. 4 shows one embodiment of creating barbs using ultrasonic energy. An apparatus 50 is used to apply ultrasonic energy to a blank workpiece (not shown). Apparatus 50 includes an ultrasonic generator 52 for energy supply, knife or blade 54 for cutting (optionally with a converter, amplifier/horn), optional rotational motor 56, optional blank workpiece gripper 58, optional light source 60, optional camera 62, optional xyz slide 64, and optional knife positioning slide 66. In embodiments, knife 54 is brought into contact with blank workpiece (not shown), wherein ultrasonic energy may be applied to the workpiece through using a converter 54 to communicate ultrasonic energy to a horn 54, causing mechanical displacement of a knife 54. In one embodiment, the knife, horn and converter are coupled together, as shown in FIG 4.

The barbs can be arranged in any suitable pattern, for example helical, linear, or randomly spaced. The number, configuration, spacing and surface area of the barbs can vary depending upon the tissue in which the suture is used, as well as the composition and geometry of the material utilized to form the suture. For example, if the wound closure device is intended to be used in fatty tissue, which is relatively soft, the barbs may be longer and spaced further apart to enable to suture or mesh to grip the soft tissue. The barbs can be arranged in various directions at various angles. In some embodiments, the filament-reinforced composite fiber may include a staggered arrangement of large or small barbs. In other embodiments, a bidirectional filament-reinforced composite fiber suture may have a random configuration of both large and small barbs.

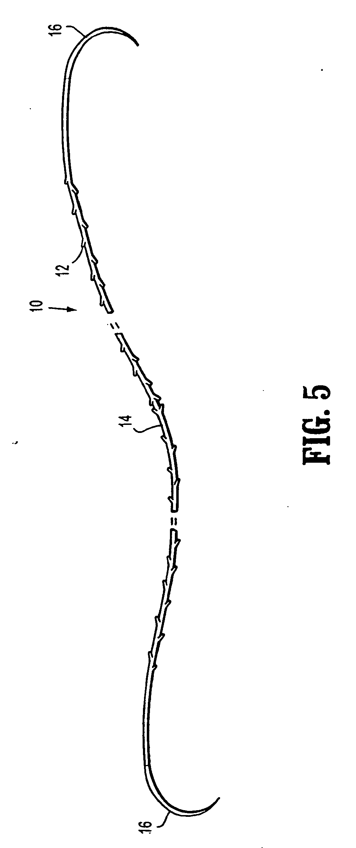

The surface area of the integral barbs can also vary. For example, fuller-tipped barbs can be made of varying sizes designed for specific surgical applications. When joining fat and relatively soft tissues, larger barbs may be desired, whereas smaller barbs may be more suitable for collagen-dense tissues. In some embodiments, a combination of large and small barbs within the same structure may be beneficial, for example when a fiber is used in tissue repair with differing layer structures. Use of the combination of large and small barbs with the same fiber wherein barb sizes are customized for each tissue layer will ensure maximum anchoring properties. In embodiments a filament-reinforced composite fiber as depicted in Figure 5 may have both large and small barbs.

In embodiments, all of the barbs may be aligned to allow the suture to move through tissue in one direction and resist moving through tissue in another direction. For example, referring to FIG. 5, the barbs 12 on a filament-reinforced composite fiber 10 may be formed into a single directional barbed suture. In embodiments, the filament-reinforced composite fiber suture 10 may be attached to needle 16. The barbs 12 are yieldable toward the body 14 of suture 10. The barbs 12 permit movement of suture 10 through tissue in the direction of movement of a needle 16 but are generally rigid in an opposite direction and prevent movement of suture 10 in a direction opposite the direction of movement of a needle 16.

In other embodiments, the barbs may be aligned on a first portion of a suture to allow movement of a first end of the suture through tissue in one direction, while barbs on a second portion of the length of the suture may be aligned to allow movement of the second end of the suture in an opposite direction. For example, as depicted in Fig. 5, a barbed suture 10 may be bidirectional. The bidirectional multifilament-reinforced composite fiber may have a needle disposed at one or both ends.

In order to facilitate needle attachment to a fiber of the present disclosure, conventional tipping agents can be applied to the braid. Two tipped ends of the fiber may be desirable for attaching a needle to each end of the fiber to provide a so-called double armed suture. The needle attachment can be made by any conventional method such as crimping, swaging, etc, as is known within the purview of those skilled in the art. Alternatively, a reduced diameter may be provided at the end of the suture to be inserted into the drilled end of a needle. To provide a reduced diameter, the suture may by machined using any technique within the purview of those skilled in the art, such as cutting, grinding, laser machining or the like.

Tissue may be sutured by passing a needled suture made from the present filament-reinforced composite fiber through tissue to close a wound. The needle may then be removed from the suture and optionally a knot tied therein. The filament-reinforced composite fiber may remain in the tissue and help prevent contamination and infection. As used herein, the term "tissue" includes, but is not limited to, tissues such as skin, fat, fascia, bones, muscles, tendons, ligaments, organs, nerves, and blood vessels. Also used herein, the term "wound" includes, but is not limited to, a surgical incision, cut, laceration or severed tissue in human or animal skin or other human or animal bodily tissue.

Filament-reinforced composite fibers of the present disclosure may be employed in medical devices, drug delivery devices and coatings. Examples of medical devices and/or surgical devices employing the filament-reinforced composite fibers may include, but are not limited to sutures, meshes, clips and other fasteners, wound dressings, bandages, drug delivery devices, anastomosis rings, stents, grafts, catheters, soft tissue repair and augmentation devices, scaffolds, buttresses, lap bands, tapes, anchors, ribbons, orthopedic devices, tissue engineering scaffolds, various cell growth substrates, and other implantable devices. In embodiments, filament-reinforced composite fibers of the present disclosure may be knitted or woven with other filaments, either absorbable or non-absorbable filaments, to form surgical devices. The filament-reinforced composite fibers also can be made into meshes or non-woven materials to form fabrics, such as matted fabrics and felts.

Additionally, the present filament-reinforced composite fibers, may be formed into a textile, such as mesh, by braiding, weaving, knitting, matting, spraying, etc., individual filaments together. FIG. 1 shows one embodiment, a mesh 30 formed from filament-reinforced composite fibers of the present disclosure. The mesh 30 has barbs 32 along a portion of the fiber-reinforced composite fiber, whereas the center of the mesh includes an unbarbed portion 36.

Additionally, the filament-reinforced composite fiber of the present disclosure may be packaged using materials known to those within the purview of those skilled in the art, including foil and various plastics ( e.g. polyethylene), which may provide a moisture barrier.

Once the filament-reinforced composite fiber is constructed, it can be sterilized by any means within the purview of those skilled in the art including but not limited to ethylene oxide, electron beam (e-beam), gamma irradiation, autoclaving, and the like.

### Example 1

Polysorb™ multifilament absorbable sutures were dipped into octyl cyanoacrylate. The cyanoacrylate was allowed to cure, providing a filament-reinforced composite fiber suture. The cyanoacrylate penetrated between the interstices of the braided suture such that the filaments of the braid are embedded within the cyanoacrylate matrix material, bound together, creating a coherent composite fiber. Barbs were manually cut along the filament-reinforced composite fiber regular intervals using a knife blade.

### Example 2

Polysorb™ multifilament absorbable sutures were dipped into a 2 wt % triclosan solution in methylene chloride. Sutures were allowed to dry, and the methylene chloride was solvent evaporated, resulting in a triclosan deposition on the braided suture. Sutures were then dipped into octyl cyanoacrylate. The cyanoacrylate was allowed to cure, providing a filament-reinforced composite fiber suture. The cyanoacrylate penetrated between the interstices of the braided suture such that the filaments of the braid are embedded within the cyanoacrylate matrix material, bound together, creating a coherent composite fiber. Barbs were manually cut along the composite multifilament suture at regular intervals using a knife blade. Samples were subject to further testing including zone of inhibition (ZOI), where the filament-reinforced composite fiber exhibited a ZOI.

It should be noted that the present disclosure is not limited to wound closure and contemplates other procedures such as cosmetic and orthopaedic procedures. Additionally, the above description contains many specifics; these specifics should not be construed as limitations on the scope of the disclosure herein but merely as exemplifications of particularly useful embodiments thereof. Those skilled in the art will envision many other possibilities within the scope of the disclosure as defined by the claims appended hereto.

Filament-reinforced composite fibers made from a plurality of filaments and a matrix are useful in forming medical devices such as sutures and meshes.

## Claims

1. A filament-reinforced composite fiber, comprising a plurality of filaments and an adhesive matrix material and at least one integral barb formed on a surface thereon, **characterised in that** the adhesive matrix material penetrates the plurality of filaments deeper than the depth of the barb.

2. The filament-reinforced composite fiber of claim 1, wherein the adhesive matrix material comprises at least 5% of a total length of the filament-reinforced composite fiber.

3. A mesh, comprising the filament-reinforced composite fiber of claim 1.

4. The filament reinforced composite fiber according to claim 1, wherein the at least one of the plurality of filaments is absorbable.

5. The filament-reinforced composite fiber according to claim 4, wherein at least one of the plurality of filaments comprises a polymer selected from the group consisting of lactide, glycolide, caprolactone, valerolactone, trimethylene carbonate, 1,4-dioxanone, σ-valeroactone, ε-caprolactone, 1,4-dioxepan-2-one, 1,5-dioxepan-2-one, collagen, gut, polymer drugs, ethylene glycol, ethylene oxide, esteramides, γ-hydroxyvalerate, β-hydroxypropionate, alpha-hydroxy acid, and β-hydroxybuterate, collagen, cellulose, gut, and copolymers thereof.

6. The filament-reinforced composite fiber according to claim 1, wherein at least one of the plurality of filaments is non-absorbable.

7. The filament-reinforced composite fiber according to claim 6, wherein at least one of the plurality of filaments comprises a polymer selected from the group consisting of silk, cotton, rubber, nylon, polypropylene, polyethylene, ultra high molecular weight polyethylene (UHMWPE), poly ethylene terepththalate, (PET), and polyesters and copolymers thereof.

8. The filament-reinforced composite fiber according to claim 1, wherein the adhesive matrix material is selected from the group consisting of monomers, oligomers, polymers.

9. The filament-reinforced composite fiber according to claim 1, wherein the adhesive matrix material comprises a polymer formed from at least one monomer selected from the group consisting of cyanoacrylates, urethanes, acrylates, fibrins, albumins, thrombins, gelatins, proteins, polysaccharides, lactones, collagens, and carbonates.

10. The filament-reinforced composite fiber according to claim 8, wherein the matrix material comprises at least one cyanoacrylate selected from the group consisting of methyl cyanoacrylate, ethyl cyanoacrylate, butyl cyanoacrylate, octyl cyanoacrylate, isobutyl cyanoacrylate and methoxypropyl cyanoacrylate and copolymers thereof.

11. The filament-reinforced composite fiber of claim 1, wherein the filament-reinforced composite fiber further comprises a medicinal agent.

12. The filament-reinforced composite fiber of claim 1 wherein the filament-reinforced composite fiber further comprises a fatty acid ester.

13. The filament-reinforced composite fiber of claim 1 wherein the filament-reinforced composite fiber further comprises a coating selected from the group consisting of polyethylene oxide, polypropylene oxide, lactide, glycolide, and caprolactone and copolymers thereof.

14. The filament-reinforced composite fiber of claim 1 wherein the filament-reinforced composite fiber is a clip, fastener, would dressing, bandage, drug delivery device, anastomosis ring, stent, graft, catheter, tissue scaffold, buttress, pledget, lap band, tape, or ribbon.

15. A suture comprising the filament-reinforced composite fibre of any of Claims 1, 2 and 4 to 14.

16. The suture of claim 15 further comprising at least one needle.

17. A medical device comprising the suture of Claim 15 or 16.

18. The medical device of claim 17 wherein the adhesive matrix material comprises 5% to 95% of the total length of the filament-reinforced composite fiber.

19. The medical device of claim 17 wherein the adhesive matrix material comprises 20% to 70% of the total length of the filament-reinforced composite fiber;

20. The medical device of claim 17 wherein the filament-reinforced composite fiber is comprised of a shape-memory polymer.

21. A method for making a medical device comprising the steps of:
applying an adhesive matrix material to a plurality of filaments to provide a filament-reinforced composite fiber; and, creating at least one barb on a surface of the filament-reinforced composite fiber, wherein the adhesive matrix material penetrates the plurality of filaments deeper than the depth of the barb.

## Patentansprüche

1. Filamentverstärkte Verbundfaser, die mehrere Filamente und ein Klebstoffmatrixmaterial und mindestens einen integralen Widerhaken, der auf einer Oberfläche darauf ausgebildet ist, umfasst, **dadurch gekennzeichnet, dass** das Klebstoffmatrixmaterial die mehreren Filamente tiefer als die Tiefe des Widerhakens durchdringt.

2. Filamentverstärkte Verbundfaser nach Anspruch 1, wobei das Klebstoffmatrixmaterial mindestens 5 % einer Gesamtlänge der filamentverstärkten Verbundfaser umfasst.

3. Netzgewebe, das die filamentverstärkte Verbundfaser nach Anspruch 1 umfasst.

4. Filamentverstärkte Verbundfaser nach Anspruch 1, wobei das mindestens eine der mehreren Filamente resorbierbar ist.

5. Filamentverstärkte Verbundfaser nach Anspruch 4, wobei mindestens eines der mehreren Filamente ein Polymer umfasst, das aus der Gruppe bestehend aus Lactid, Glycolid, Caprolacton, Valerolacton, Trimethylencarbonat, 1,4-Dioxanon, σ-Valerolacton, ε-Caprolacton, 1,4-Dioxepan-2-on, 1,5-Dioxepan-2-on, Collagen, Darm, Polymerwirkstoffen, Ethylenglycol, Ethylenoxid, Esteramiden, γ-Hydroxyvalerat, β-Hydroxypropionat, alpha-Hydroxysäure und β-Hydroxybutyrat, Collagen, Cellulose, Darm und Copolymeren davon ausgewählt ist.

6. Filamentverstärkte Verbundfaser nach Anspruch 1, wobei mindestens eines der mehreren Filamente nicht resorbierbar ist.

7. Filamentverstärkte Verbundfaser nach Anspruch 6, wobei mindestens eines der mehreren Filamente ein Polymer umfasst, das aus der Gruppe bestehend aus Seide, Baumwolle, Kautschuk, Nylon, Polypropylen, Polyethylen, ultrahochmolekularem Polyethylen (UHMWPE), Polyethylenterephthalat (PET) und Polyestern und Copolymeren davon ausgewählt ist.

8. Filamentverstärkte Verbundfaser nach Anspruch 1, wobei das Klebstoffmatrixmaterial aus der Gruppe bestehend aus Monomeren, Oligomeren, Polymeren ausgewählt ist.

9. Filamentverstärkte Verbundfaser nach Anspruch 1, wobei das Klebstoffmatrixmaterial ein Polymer umfasst, das aus mindestens einem Monomer gebildet wurde, das aus der Gruppe bestehend aus Cyanoacrylaten, Urethanen, Acrylaten, Fibrinen, Albuminen, Thrombinen, Gelatinen, Proteinen, Polysacchariden, Lactonen, Collagenen und Carbonaten ausgewählt ist.

10. Filamentverstärkte Verbundfaser nach Anspruch 8, wobei das Matrixmaterial mindestens ein Cyanoacrylat umfasst, das aus der Gruppe bestehend aus Methylcyanoacrylat, Ethylcyanoacrylat, Butylcyanoacrylat, Octylcyanoacrylat, Isobutylcyanoacrylat und Methoxypropylcyanoacrylat und Copolymeren davon ausgewählt ist.

11. Filamentverstärkte Verbundfaser nach Anspruch 1, wobei die filamentverstärkte Verbundfaser weiterhin ein Arzneimittel umfasst.

12. Filamentverstärkte Verbundfaser nach Anspruch 1, wobei die filamentverstärkte Verbundfaser weiterhin einen Fettsäureester umfasst.

13. Filamentverstärkte Verbundfaser nach Anspruch 1, wobei die filamentverstärkte Verbundfaser weiterhin eine Beschichtung umfasst, die aus der Gruppe bestehend aus Polyethylenoxid, Polypropylenoxid, Lactid, Glycolid und Caprolacton und Copolymeren davon ausgewählt ist.

14. Filamentverstärkte Verbundfaser nach Anspruch 1, wobei die filamentverstärkte Verbundfaser eine Klemme, ein Befestigungsmittel, ein Wundverbandmaterial, ein Verband, eine Wirkstoffabgabevorrichtung, ein Anastomosenring, ein Stent, ein Transplantat, ein Katheter, ein Gewebegerüst, ein Stützpfeiler, ein Pledget, ein LAP-Band, eine Binde oder ein Band ist.

15. Nahtfaden, der die filamentverstärkte Verbundfaser nach einem der Ansprüche 1, 2 und 4 bis 14 umfasst.

16. Nahtfaden nach Anspruch 15, der weiterhin mindestens eine Nadel umfasst.

17. Medizinische Vorrichtung, die den Nahtfaden nach Anspruch 15 oder 16 umfasst.

18. Medizinische Vorrichtung nach Anspruch 17, wobei das Klebstoffmatrixmaterial 5 % bis 95 % der Gesamtlänge der filamentverstärkten Verbundfaser umfasst.

19. Medizinische Vorrichtung nach Anspruch 17, wobei das Klebstoffmatrixmaterial 20 % bis 70 % der Gesamtlänge der filamentverstärkten Verbundfaser umfasst.

20. Medizinische Vorrichtung nach Anspruch 17, wobei die filamentverstärkte Verbundfaser aus einem Formgedächtnispolymer zusammengesetzt ist.

21. Verfahren zur Herstellung einer medizinischen Vorrichtung, das die folgenden Schritte umfasst:
Aufbringen eines Klebstoffmatrixmaterials auf mehrere Filamente, um eine filamentverstärkte Verbundfaser bereitzustellen; und Erzeugen mindestens eines Widerhakens auf einer Oberfläche der filamentverstärkten Verbundfaser, wobei das Klebstoffmatrixmaterial die mehreren Filamente tiefer als die Tiefe des Widerhakens durchdringt.

## Revendications

1. Fibre composite renforcée de filaments, comprenant une pluralité de filaments et une matière matricielle adhésive et au moins un picot d'un seul tenant formé sur une surface de celle-ci, **caractérisée en ce que** la matière matricielle adhésive pénètre dans la pluralité de filaments plus profond que la profondeur du picot.

2. Fibre composite renforcée de filaments selon la revendication 1, dans laquelle la matière matricielle adhésive constitue au moins 5 % d'une longueur totale de la fibre composite renforcée de filaments.

3. Treillis, comprenant la fibre composite renforcée de filaments selon la revendication 1.

4. Fibre composite renforcée de filaments selon la revendication 1, dans laquelle l'au moins un filament de la pluralité de filaments peut être absorbé.

5. Fibre composite renforcée de filaments selon la revendication 4, dans laquelle au moins un filament de la pluralité de filaments comprend un polymère sélectionné à partir du groupe constitué par le lactide, le glycolide, la caprolactone, la valérolactone, le carbonate de triméthylène, le 1,4-dioxanone, la σ-valérolactone, l'ε-caprolactone, le 1,4-dioxépan-2-one, le 1,5-dioxépan-2-one, le collagène, le boyau, des médicaments polymères, l'éthylène glycol, l'oxyde d'éthylène, des estéramides, le γ-hydroxyvalérate, le β-hydroxypropionate, l'acide alpha-hydroxy et le β-hydroxybutyrate, le collagène, la cellulose, le boyau, et des copolymères de ceux-ci.

6. Fibre composite renforcée de filaments selon la revendication 1, dans laquelle au moins un filament de la pluralité de filaments ne peut pas être absorbé.

7. Fibre composite renforcée de filaments selon la revendication 6, dans laquelle au moins un filament de la pluralité de filaments comprend un polymère sélectionné à partir du groupe constitué par la soie, le coton, le caoutchouc, le nylon, le polypropylène, le polyéthylène, le polyéthylène de masse moléculaire ultra-élevée (UHMWPE), le polytéréphtalate d'éthylène, (PET), et des polyesters et des copolymères de ceux-ci.

8. Fibre composite renforcée de filaments selon la revendication 1, dans laquelle la matière matricielle adhésive est sélectionnée à partir du groupe constitué par des monomères, des oligomères, des polymères.

9. Fibre composite renforcée de filaments selon la revendication 1, dans laquelle la matière matricielle adhésive comprend un polymère formé à partir d'au moins un monomère sélectionné à partir du groupe constitué par des cyanoacrylates, des uréthanes, des acrylates, des fibrines, des albumines, des thrombines, des gélatines, des protéines, des polysaccharides, des lactones, des collagènes et des carbonates.

10. Fibre composite renforcée de filaments selon la revendication 8, dans laquelle la matière matricielle comprend au moins un cyanoacrylate sélectionné à partir du groupe constitué par le cyanoacrylate de méthyle, le cyanoacrylate d'éthyle, le cyanoacrylate de butyle, le cyanoacrylate d'octyle, le cyanoacrylate d'isobutyle et le cyanoacrylate de méthoxypropyle et des copolymères de ceux-ci.

11. Fibre composite renforcée de filaments selon la revendication 1, dans laquelle la fibre composite renforcée de filaments comprend en outre un agent médicinal.

12. Fibre composite renforcée de filaments selon la revendication 1, dans laquelle la fibre composite renforcée de filaments comprend en outre un ester d'acide gras.

13. Fibre composite renforcée de filaments selon la revendication 1, dans laquelle la fibre composite renforcée de filaments comprend en outre un revêtement sélectionné à partir du groupe constitué par l'oxyde de polyéthylène, l'oxyde de polypropylène, le lactide, le glycolide, et la caprolactone et des copolymères de ceux-ci.

14. Fibre composite renforcée de filaments selon la revendication 1, dans laquelle la fibre composite renforcée de filaments est une agrafe, une attache, du baume pour blessure, un bandage, un dispositif de délivrance de médicament, une bague d'anastomose, une endoprothèse, un greffon, un cathéter, un échafaudage de tissu, un contrefort, une compresse, une bande de recouvrement, une bande, ou un ruban.

15. Suture comprenant la fibre composite renforcée de filaments selon l'une quelconque des revendications 1, 2 et 4 à 14.

16. Suture selon la revendication 15 comprenant en outre au moins une aiguille.

17. Dispositif médical comprenant la suture selon la revendication 15 ou 16.

18. Dispositif médical selon la revendication 17, dans lequel la matière matricielle adhésive constitue 5 % à 95 % de la longueur totale de la fibre composite renforcée de filaments.

19. Dispositif médical selon la revendication 17, dans lequel la matière matricielle adhésive constitue 20 % à 70 % de la longueur totale de la fibre composite renforcée de filaments.

20. Dispositif médical selon la revendication 17, dans lequel la fibre composite renforcée de filaments est constituée par un polymère à mémoire de forme.

21. Procédé pour fabriquer un dispositif médical comprenant les étapes de :
application d'une matière matricielle adhésive à une pluralité de filaments pour réaliser une fibre composite renforcée de filaments ; et, création d'au moins un picot sur une surface de la fibre composite renforcée de filaments, dans laquelle la matière matricielle adhésive pénètre dans la pluralité de filaments plus profond que la profondeur du picot.
